# EUROPEAN PATENT APPLICATION

(11) **EP 1 577 375 A2**
(43) Date of publication of application: **21.09.2005**
(21) Application number: 05005446.9
(22) Date of filing: 12.03.2005
(51) Int. Cl.: C11D 17/04, C11D 17/08, A61K 7/00, A61K 7/48

(54) **Hand soap product and hand soap dispenser**

(30) Priority: 18.03.2004 DK 200400442; 16.06.2004 US 870331
(71) Applicant: Jahnsen, Torben Reindahl, Kobenhavn K 1400 (DK)
(72) Inventor: Jahnsen, Torben Reindahl, Kobenhavn K 1400 (DK)
(74) Representative: van Walstijn, Bartholomeus G. G.

(57) **Abstract**

A hand soap product in the form of water dissolvable capsules filled with liquid hand soap. A dispenser and method for dispensing the water dissolvable capsules, and a dispenser for spherical solid soap tablets. A fizzing hand soap tablet.

It is suggested that Fig. 1 is published with the abstract.

## Description

### INTRODUCTION

The present invention relates to hand care products. More particularly, the invention relates to liquid hand soap in a water dissolvable capsule, and a dispenser for dispensing the hand soap filled water dissolvable capsules. The invention also relates to a dispenser for spherical soap tablets.

### BACKGROUND OF THE INVENTION

Contemporary standards of personal hygiene require regular cleaning of the hands. Modern hand soap compositions effectively clean the hands, by removing excess soil and sebum, and can also include additional ingredients such as moisturizing agents.

Usually, liquid hand soaps contain a detergent, a primary lathering agent, a thickening agent and an opacifying agent in a water carrier as the principal ingredients. An exemplary hand soap composition is disclosed by Shields, U.S. Pat. No. 4,617,148.

Hand soap compositions have hitherto been formulated for packaging and dispensing from conventional containers, such as plastic bottles with pump actuated dispensers, tubes, etc. An exemplary manual pump actuated hand soap dispenser is disclosed by Bitton, U.S. Pat. No. 5,915,600.

A disadvantage of manual pumps can though be the need to touch and the risk of contamination via the surface of the pump.

From a hygienic standpoint automatic dispensers with a touchless activation are preferred. An exemplary touchless automatic hand soap dispenser is disclosed by Mease et al., U.S. 5.255.822.

A disadvantage associated with automatic liquid hand soap dispensers is spilled soap that accumulates under the dispenser caused by soap still dripping from the dispenser when the user has already withdrawn his hand. The relatively high viscosity of the liquid soap renders it practically impossible to cut the flow of soap instantaneously, hence the large risk of a user not waiting until the dispenser has completely stopped dripping soap. The area around the discharge opening of the dispenser also tends to accumulate soap rests and requires regular cleaning.

Tablets of solid soap are an alternative to liquid soap dispensers. An exemplary soap tablet dispenser is disclosed by Satoshi, JP 01-259821, for dispensing solid spherical soap spheres. This dispenser is manually operated, with the hygienic disadvantages of contamination via touching of the components of the dispenser as discussed above.

A need exists for a hand soap dispenser and a hand soap therefore that are completely hygienic and reduce spilling of soap.

### SUMMARY

The invention described below provides a hand care product, a dispenser, or dispensing system or method for dispensing hand care or like products.

The hand care product may be a liquid hand soap composition that is encapsulated in a water dissolvable shell. The shell can be made from a gelatin based product.

The dispenser may comprise a chamber for receiving a plurality of water dissolvable substantially spherical capsules filled with hand soap, and a mechanism for releasing one capsule at a time.

According to an embodiment, the mechanism can be hand activated.

According to another embodiment, the mechanism can be electrically operated. The electrically operated mechanism may be activated by a touchless sensor.

According to a further embodiment, the mechanism may comprise a sliding member disposed within a downward path leading from a lowest point in the chamber to an exit.

According to an embodiment, the mechanism comprises a rotatable member disposed within a downward path leading from a lowest point in the chamber to an exit. The rotatable member may comprises a hollow hemisphere or hollow spherical cap configured to receive one capsule or spherical soap tablet at a time, the hollow hemisphere or hollow spherical cap being rotatably suspended about an axis traverse to the extension of the path.

Preferably, one capsule or spherical tablet is released from the dispenser for each full revolution of the rotatable member.

According to yet another embodiment the dispenser may comprise a chamber for receiving a plurality of spherical solid soap tablets, an electrically operated mechanism for releasing one spherical solid soap tablet at a time, detection circuitry operative to detect presence of a user's hand at a predetermined location near said dispenser; a rotatable member disposed within a downward path leading from a lowest point in said chamber to an exit; a hollow hemisphere or hollow spherical cap disposed in said path and configured to receive one capsule or spherical soap tablet at a time, said hollow hemisphere or hollow spherical cap being rotatably suspended about an axis traverse to the extension of said path and actuated by said electrically operated mechanism.

The method of dispensing hand soap may comprise the steps of providing hand soap in water dissolvable capsules, placing the capsules in a chamber inside a dispenser and dispensing a prescribed amount of capsules at a time.

### BRIEF DESCRIPTION OF THE DRAWINGS

In the following detailed portion of the present description, some embodiments of the invention will be explained in more detail with reference to the exemplary embodiments shown in the drawings, in which:
Fig. 1 is a cross-sectional view of the dispenser with the rotatable member in an idle position;
Fig. 2 is a bottom view of the dispenser;
Fig. 3 is a cross-sectional view of the dispenser with the rotatable member in a first non-idle position;
Fig. 4 is a cross-sectional view of the dispenser with the rotatable member in a second non-idle position;
Fig. 5 is a block diagram of the controls of the dispenser.
Fig .6 is a cross-sectional view of another preferred embodiment of the dispenser; and
Fig. 7 is a top view of a bottom part of the dispenser shown in Fig. 6.

### DETAILED DESCRIPTION OF THE INVENTION

The present invention is generally directed to dispensing apparatuses, systems and/or methods for dispensing hand soap or like products.

Generally, a plurality of spheres of such products is disposed in a dispenser as described hereinbelow which has an opening through which the products are dispensed. The spheres are according one preferred embodiment formed by water dissolvable capsules filled with liquid hand soap.

Hereafter, the term "hand soap" will be used to define the types of compositions to be dispensed in the water dissolvable capsules. Hand soap therefore encompasses liquid soap as understood in the art, e.g. for hygienic or medical or other uses; and hand soap also encompasses the like or other combination or substitute material products which can be used herein, as for example surfactants, e.g. sodium laureth sulfate, sodium lauryl sulfate, ammonium lauryl sulfate, inter alia, thickeners such as hydroxyethyl cellulose or hydroxypropyl guar, inter alia and blends thereof.

Hereafter, the term "solid soap" will be used to define the types of compositions to be dispensed as solid spheres. Solid soap therefore encompasses solid soap as understood in the art, e.g. for hygienic or medical or other uses.

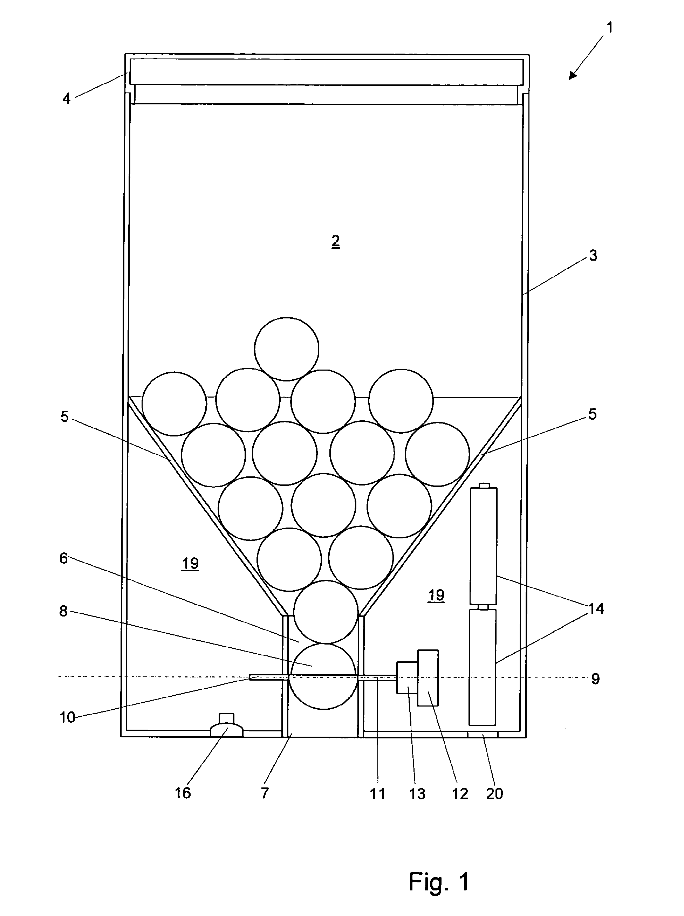

Fig. 1 shows a cross-sectional view of a preferred embodiment of a dispenser 1 having a generally cylindrical shape. An internal chamber 2 is formed in the upper part of the housing 3.

The top of the housing 3 is covered by a lid 4 that can be removed for filling the chamber 2 with hand care products in the form of spherical solid soap tablets or in the form of water dissolvable spherical capsules filled with liquid hand soap.

The lid 4 is according to a preferred embodiment provided with locking means, for which e.g. a key (not shown) is required. A heavy plate or other heavy object (not shown) may be placed on top of the spheres in internal the chamber 2 to push the spheres down.

The chamber 2 is provided with a tapered and preferably conical bottom 5 that leads to a downward path 6 leading to an exit 7 from which the spherical capsules or spherical tablets can leave the housing. A rotatable substantially hollow hemispherical member 8 is disposed in the path 6.

The rotatable member 8 is rotatably suspended along an axis 9 substantially traverse to the extension of the downward path 6. Shafts 10,11 extend along the axis 9 from rotatable member 8 in two opposite directions. The shaft 10 is journalled in a recess in the surface of the path 6.

On the opposite side the shaft 11 is journalled in a bore in wall of the path 6, and connected to an electromagnetic actuator. In a preferred embodiment the shaft 11 is connected to a rotary electric motor, preferably a stepper motor 12, via a reduction gearing 13.

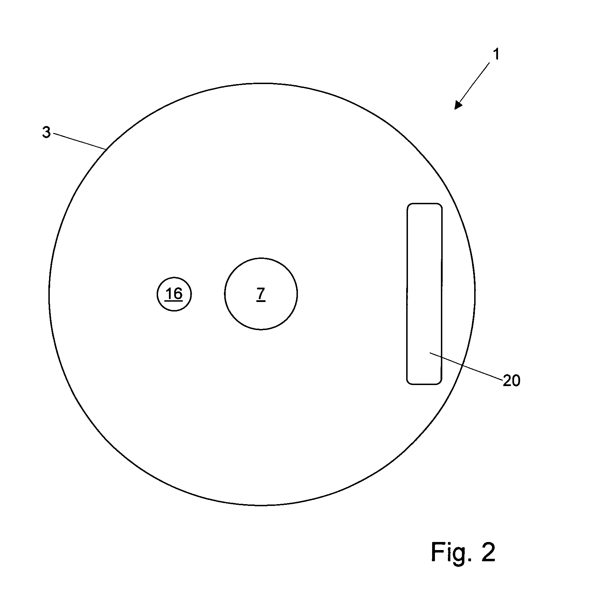

The stepper motor is powered by a battery unit 14 and controlled by a controller unit 15. As shown in fig. 2, access to the batteries is possible via lid 20 in the bottom of the housing 1.

The space 19 under the conical bottom 5 surrounding the stepper motor 12, the gearing 13, and the batteries 14 is filled with a suitable material such as plastic foam.

A touchless sensor 16 disposed in the bottom of the housing is connected to the controller unit 15.

In presently preferred embodiments, the detection circuitry utilizes the optical reflectivity of the user's hand to detect its presence near dispenser 1. It should be distinctly understood, however, that various types of active and passive proximity detection circuits may be utilized for this purpose, such as infrared sensors.

The inner radius of the rotatable member 8 is substantially equal to or somewhat overdimensioned with respect to the spherical capsules and the spherical tablets.

In the idle position shown in Fig. 1 a single spherical capsule or spherical tablet is received in the rotatable member 8. When a user places a hand under the dispenser 1 the touchless sensor 16 sends a signal to the controller unit 15. Upon receiving the signal form the touchless sensor 16 the controller unit 15 instructs the stepper motor 12 perform a rotational movement that corresponds to one revolution (360° turn) of the rotatable member 8. The rotational movement of the stepper motor 12 equals 360° multiplied by the gearing ration of the gearing 13.

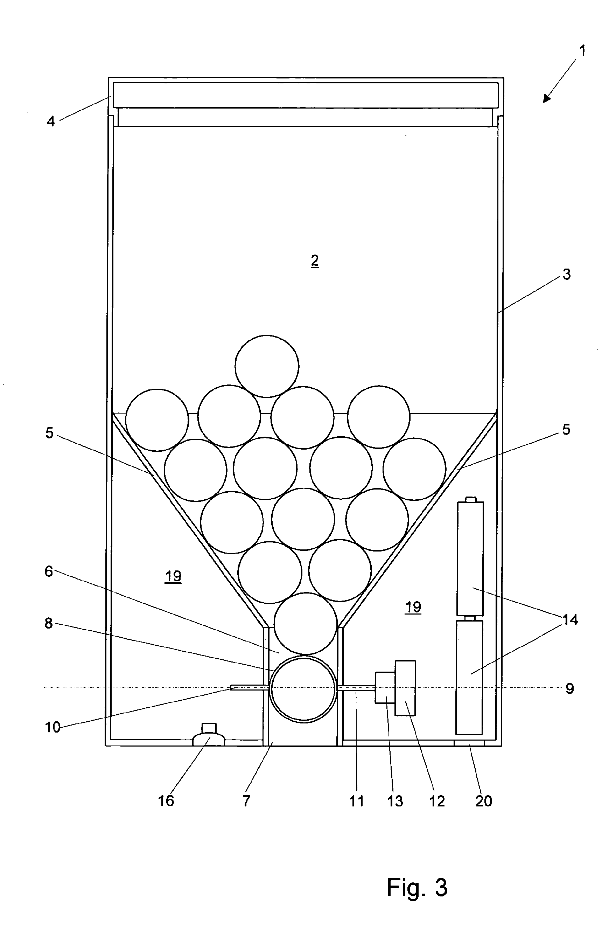

Fig. 3 shows the rotatable member 8 after is has completed 90° of the 360° turn.

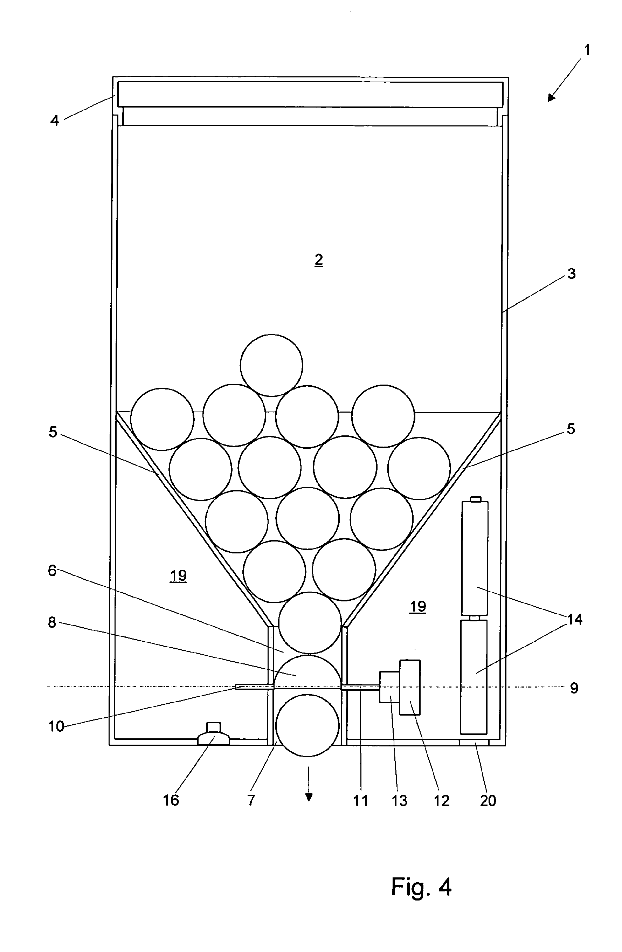

At the point where the rotatable member 8 has, as shown in Fig. 4 turned 180°, the spherical capsule or spherical tablet first received in the rotatable member 8 is released and under the influence of the gravitation pull it leaves the path 6 via exit 7 and falls into the hand of the user (not shown). During the next 180° of the revolution the rotatable member receives a new spherical capsule or spherical tablet and resumes the idle position of Fig. 1, ready for dispensing another capsule or spherical tablet.

The spherical capsules comprise a hand soap with a relatively low water content, the equilibrium relative humidity (ERH) preferably being less than about 65%. The ERH is a measure of the amount of water in a composition that is free, or "active". In most compositions containing water, at least a portion of the water molecules is strongly bound to various sites, in particular polar sites, on the various chemical constituents of the composition. Such sites include hydroxyl groups, carbonyl groups, amino groups, and other sites that are capable of binding water by hydrogen bonding, ionic bonding, etc. Additional quantities of water molecules may be bound less strongly, yet still be effectively unavailable as a solvent for the composition or for materials with which the composition comes into contact. The remaining water is unbound, that is, free.

The ERH of a composition is measured by determining the humidity, in an enclosed volume, at which the vapor pressure of water contained in the composition is equal to the vapor pressure of water in the volume of air above the composition. ERH, and the related quantity of water activity (obtained by dividing the measured ERH by 100), can readily be measured using instruments available commercially from, e.g., Rotronic Instrument Corp. (Huntington, N.Y.). Various methods for measuring ERH and water activity are described in R. Marsili, "Water Activity: Why It's Important and How to Measure It," Food Product Design, December 1993, pp. 36-41, which is incorporated herein by reference.

Compositions having an ERH of less than about 65%, more preferably between about 65% and 35%, have proven suitable for encapsulation according to the invention and are therefore preferred.

According to another preferred embodiment, the spherical tablets are fizzing solid soap tablets. These tables contain solid soap and a fizzing agent to assist in accelerating the dissolving process when the tablets come in contact with water. The fizzing agent can be any conventional fizzing agent as known from e.g. medicinal fizzing tablets. Suitable fizzing agents may contain carbonate such as Calcium Carbonate (CaCO3), Calcium Bicarbonate (Ca(HCO3)2)or Sodium Bicarbonate (NaHC03) and an acidic component. Suitable acidic components are e.g. citric acid (C6H8O7)and Acetic Acid (HC2H3O2).

In a preferred embodiment, the spherical capsules have diameter in the range of 7 to 14 mm.

In a preferred embodiment, the spherical tablets have diameter in the range of 3 to 12 mm.

The tablets may comprise solid soap, or pressed soap powder with or without binder or hard gel type hand soap.

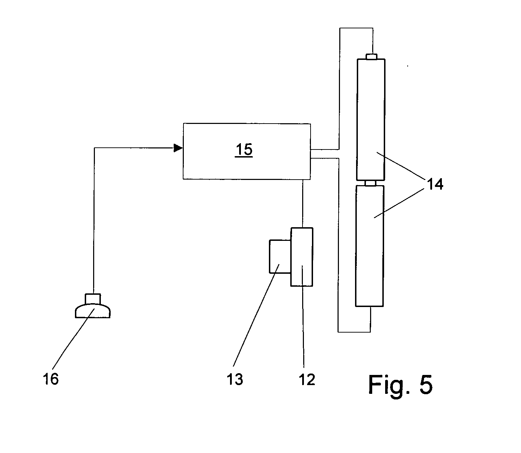

Fig. 5 shows a block diagram of the compounds of the dispensing control system. The battery unit 14 is connected to a control unit 15. The control unit 15 is connected to the touchless sensor 16 and to the stepper motor 12. The control unit 15 can be a microprocessor or a more simple electric circuit.

The control unit 15 is configured to instruct the stepper motor 12 to complete a given angular distance that corresponds to one revolution of the rotatable member 8 each time it receives a signal form the touchless sensor 16. Thus, a single sphere is dispensed for every activation of the touchless sensor 16. The control unit 15 can be configured to apply a delay before releasing a next sphere, in order to prevent fast repetitive releasing of spheres.

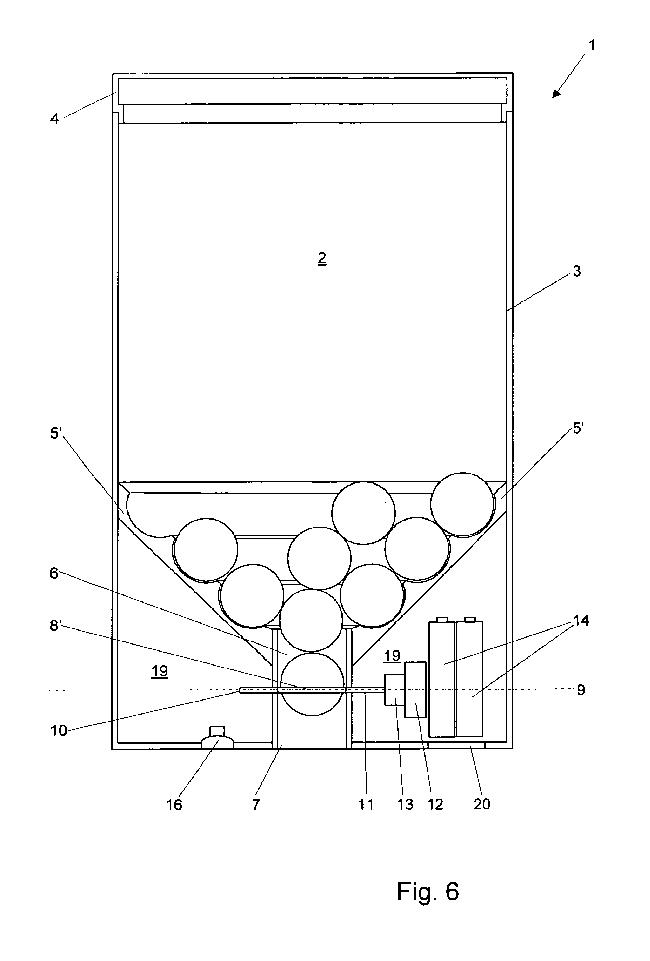

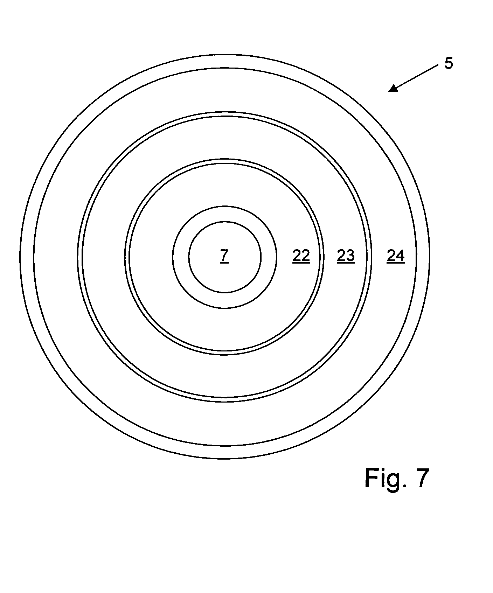

Figs. 6 and 7 disclose another preferred embodiment of the invention that is equal to the embodiments of Figs. 1 to 5 with the following changes. The conical bottom 5 is undulated to provide three circular tracks that avoids that the spheres assume a configuration in which they lock one another and thus obstruct the dispensing of spheres. The outermost track 24 is configured to hold twenty-three spheres. The middle track 23 is configured to hold fifteen spheres an the innermost track 21 is configured to hold seven spheres. This arrangement for the conical bottom 5 avoids sphere lockup, e.g. a situation in which the spheres do not enter the downward path 6 since their interaction prevents the lowest sphere from falling into the downward path 6. It is understood that arrangements that accommodate other numbers of spheres than described above can also be used and can be selected in accordance with the size of the spheres and of the container and the convergence angle of the funnel shaped section.

The hollow hemispherical member 8 is replaced with a ring 8' with an inner diameter that is slightly smaller than the outer diameter of the spheres. A sphere can thus be received in the ring 8' was shown in Fig. 6 without falling trough the ring 8'. A 90° turn of the shaft 11,10 results in the simultaneous discharge of a sphere and loading of new sphere in the ring 8', ready to be discharged. The downward path 6 is slightly enlarged to make place for a sphere that is being rotated together with the ring 8'.

In a not shown embodiment the dispenser mechanism comprises a sliding member disposed within the downward path leading from the lowest point in chamber to the exit.

While the preferred embodiments of the product, device and method have been described in reference to the environment in which they were developed, they are merely illustrative of the principles of the invention. Other embodiments and configurations may be devised without departing from spirit of the invention and the scope of the appended claims.

## Claims

1. A hand care product comprising a liquid hand soap composition encapsulated in a water dissolvable shell.

2. A hand care product according to claim 1, wherein said hand soap composition has an equilibrium relative humidity of less than 65%.

3. A dispenser for dispensing hand soap comprising a chamber for receiving a plurality of water dissolvable substantially spherical capsules filled with hand soap, and a mechanism for releasing one capsule at a time.

4. A dispenser according to claim 3, wherein the mechanism is electrically operated.

5. A dispenser according to claim 4, further comprising detection circuitry operative to detect presence of a user's hand at a predetermined location near said dispenser.

6. A dispenser according to any of claims 3 to 5, wherein the mechanism comprises a rotatable member disposed within a downward path leading from a lowest point in said chamber to an exit.

7. A dispenser according to claim 6, wherein said rotatable member comprises a hollow hemisphere or hollow spherical cap configured to receive one capsule or spherical soap tablet at a time, said hollow hemisphere or hollow spherical cap being rotatably suspended about an axis traverse to the extension of said path.

8. A dispenser according to claim 7, wherein one capsule or spherical tablet is released from said dispenser for each full revolution of said rotatable member.

9. A dispenser for dispensing hand soap comprising:
a chamber for receiving a plurality of spherical soap tablets or capsules;
an electrically operated mechanism for releasing one spherical soap tablet or capsule at a time;
detection circuitry operative to detect presence of a user's hand at a predetermined location near said dispenser;
a rotatable member disposed within a downward path leading from a lowest point in said chamber to an exit;
a hollow hemisphere or hollow spherical cap disposed in said path and configured to receive one spherical soap tablet or capsule at a time, said hollow hemisphere or hollow spherical cap being rotatably suspended about an axis traverse to the extension of said path and actuated by said electrically operated mechanism.

10. A dispenser according to claim 19, wherein one spherical tablet or capsule is released from said dispenser for each full revolution of said rotatable member.

11. A method of dispensing hand soap comprising the steps of providing hand soap in water dissolvable capsules, placing said capsules in a chamber inside a dispenser and dispensing a prescribed amount of capsules at a time.

12. A preferably substantially spherical hand soap tablet containing a fizzing agent.

13. A hand soap tablet according to claim 15, wherein the fizzing agent contains Calcium Carbonate and/or Calcium Bicarbonate and/or Sodium Bicarbonate and an acid component.

14. A hand soap tablet according to claim 13, in which the acid component comprises citric acid and/or Acetic acid.
